Europäisches Patentamt

European Patent Office

Office européen des brevets

(19)

(11) EP 1 342 474 A1

(12) **EUROPEAN PATENT APPLICATION**

(43) Date of publication:
**10.09.2003 Bulletin 2003/37**

(51) Int Cl.⁷: **A61K 31/401**, A61K 38/00,
A61K 38/39, A61K 38/17,
A61K 7/48, A23L 3/34,
A61P 1/00, A61P 39/06,
A61P 17/00

(21) Application number: **02075964.3**

(22) Date of filing: **08.03.2002**

(84) Designated Contracting States:
**AT BE CH CY DE DK ES FI FR GB GR IE IT LI LU
MC NL PT SE TR**
Designated Extension States:
**AL LT LV MK RO SI**

(71) Applicant: **Universiteit Leiden**
**2312 AV Leiden (NL)**

(72) Inventors:
• **Alia**
**2343 PK Oegstgeest (NL)**

• **Matysik, Jörg**
**2343 PK Oegstgeest (NL)**
• **Backendorf, Claude Maria Pierre**
**2334 EM Leiden (NL)**

(74) Representative: **Prins, Adrianus Willem et al**
**Vereenigde,**
**Nieuwe Parklaan 97**
**2587 BN Den Haag (NL)**

(54) **The use of proline, poly-proline peptides and proline-rich proteins to quench reactive oxygen species**

(57)      The invention relates to the fields of human medicine, cosmetics and food, more particular to the prevention or inhibition of oxidation by quenching of radicals, in particular reactive oxygen species. The use of proline and/or derivatives thereof, and/or protein and/or peptide rich in said proline and/or derivatives thereof is claimed for the protection against damage by ROS. Pharmaceutical and topical compositions and methods to protect cells and tissues against damage by ROS are also claimed.

EP 1 342 474 A1

## Description

The invention relates to the fields of human medicine, cosmetics and food, more particular to the prevention or inhibition of oxidation by quenching of reactive oxygen species.

Less than 5% of the total pool of free amino acids in plants under stress-free conditions is provided by proline. In many plants under various forms of stress, the concentration increases up to 80% of the amino acid pool. This observation indicates that a high proline concentration is favourable under stress conditions. In addition to the role of proline as osmolyte and reservoir of carbon and nitrogen etc, proline has been shown to protect plants against free radical induced damage. Recently, we disclosed that, in a certain pathway in thylakoid membranes, proline seems to play a role in quenching of singlet oxygen, erroneously produced in said pathway (1).

Herein we disclose that ROS (and other radicals) quenching by proline, or derivatives thereof, can be used to e.g. prevent or diminish possible damage to a wide variety of substances, tissues and cells.

The present invention is based for a substantial part on the finding that ROS quenching by proline is far more wide spread than the action, disclosed by us, in the thylakoid membranes of certain plants.

We herein disclose the mechanism of action of quenching ROS and other radicals by proline or functional derivatives thereof and the use of this quenching mechanism in many different fields.

In this application, we disclose the use of proline and/or functional derivatives thereof, and/or protein and/or peptide rich in said proline and/or functional derivatives thereof for quenching radicals, in particular reactive oxygen species (ROS). Said functional proline derivative has at least in kind, if not in amount the same function of quenching radicals as proline, said quenching comprising physical and/or chemical quenching of radicals, in particular of ROS.

Quenching radicals can be useful in many applications. Chemical reactions that depend on radical initiation can be stopped. Side reactions involving radicals can be inhibited, etc.

Particularly, when radicals are unwanted, quenching according to the present invention is useful. A lot of materials, in particular biological materials, can suffer damage from radicals, in particular ROS. Because proline is safe for biological materials, and in general for quenching of ROS, it is an excellent and safe method for preventing or diminishing damage caused by radicals. Of course, derivatives, which retain the quenching function of proline (functional derivatives) such as hydroxyproline, can also be used.

Because proline or a functional derivative thereof can also be incorporated in a protein or a peptide or a derivative thereof, this application also discloses the use of proline-rich proteinaceous molecules of, for example, but not limited to the gene family of proline-rich proteins (PrP's) and/or small proline-rich proteins (SPRR) and/or Late Envelope Proteins (LEP), and/or collagen, and/or elastin, and/or prolamine, and/or casein, and/or proline-rich muscle proteins and/or polyproline peptides for preventing and/or diminishing damage by radicals, in particular ROS. Said LEP proteins (late envelope protein), are present in the skin and have an N-terminal domain (rich in proline) which is very similar to the corresponding domain in SPRR proteins (most probably because they have a common ancestor). Said proline rich proteins (PrPs) are present in e.g. saliva. The proline repeats in these proteins are different from those in SPRR and LEP, but these proteins a comparable antioxidant function as SPRR.

Thus, in one embodiment, said proline and/or functional derivatives thereof, and/or protein and/or peptide rich in said proline and/or functional derivatives thereof are used to prevent and/or diminish damage by ROS, wherein additional proline and/or functional derivatives thereof, and/or protein and/or peptide rich in said proline and/or functional derivatives thereof is provided for preventing damage to cells and tissues.

Said additional proline and/or functional derivatives thereof, and/or protein and/or peptide rich in said proline and/or functional derivatives thereof can also be used for the preparation of a medicament for diminishing or preventing damage to cells and/or tissues by ROS.

In another embodiment, said additional proline and/or functional derivatives thereof, and/or protein and/or peptide rich in said proline and/or functional derivatives thereof which are able to quench ROS, can be added to a food substance, or to a topical composition, or to a pharmaceutical composition. In food said proline and/or functional thereof, and/or protein and/or peptide rich in said proline and/or functional derivatives thereof can diminish or prevent damage by ROS to the food during storage and preparation, and also to the consumer of the food by preventing or diminishing damage by ROS to the mucosa of the digestive tract. In a pharmaceutical composition, said proline and/or functional derivatives thereof, and/or protein and/or peptide rich in said proline and/or functional derivatives thereof can diminish or prevent damage by ROS to the pharmaceutical composition during storage and preparation, and in the same or in a different composition, also to the consumer of the pharmaceutical composition by preventing or diminishing damage by ROS to the cells and tissues of the body. Therefore, the invention also provides a composition comprising proline and/or functional derivatives thereof, and/or protein and/or peptide rich in said proline and/or functional derivatives thereof.

In another embodiment, the invention provides a topical composition comprising proline and/or functional derivatives thereof, and/or protein and/or peptide rich in said proline and/or functional derivatives thereof and a suitable carrier or diluent. Said topical composition is for example a sun-screening or sun-blocking lotion and can help to avoid or diminish photosensitivity of the skin. Therefore, the invention also provides a method to prevent damage to the skin during tanning comprising applying said composition prior to exposure of the body to a ROS inducing condition, for example radiation, for example solar radiation or artificial radiation by sun-tanning apparatus. Said topical composition also comprises, cosmetic products like for example: soap, shampoo, hair dye, hair-bleaching solutions, nail-care products, and dental-care products. Therefore, said invention also provides a method to protect hair, eyes, nails, skin and teeth against damage by ROS comprising applying said composition prior to exposure of the body to a ROS inducing condition, for example staining, bleaching, or cleaning.

In another embodiment, the invention provides a pharmaceutical composition comprising a proline-rich proteinaceous molecule of the gene family of proline-rich proteins (PrP's) and/or small proline-rich proteins (SPRR) and/or Late Envelope Proteins (LEP) and/or collagen, and/or casein, and/or elastin and/or prolamine and/or muscle protein and a suitable carrier or diluent. The invention also provides a method to protect cells and tissues of a mammal against damage by ROS or other radicals by administration of said pharmaceutical composition prior to a ROS inducing treatment or diagnosis.

Food with high proline contents deposits the proline and/ or proline-rich proteins or -peptides in the digestive tract. There, said proline and or proline-rich proteins or -peptides quench topical ROS and prevent damage of the digestive tract cells and tissues. In this way, food intake with additional proline or proline-rich proteins or -peptides may prevent and/ or alleviate stomach ulcers, duodenal ulcers, and lower gut inflammation and/or chronic inflammatory reactions such as for example, but not limited to Crohn's disease. Therefore, the invention teaches the use of proline and/or functional derivatives thereof, and/or protein and/or peptide rich in said proline and/or functional derivatives thereof for the preparation of a food anti-oxidant and/or nutraceutical composition. In another embodiment, the invention provides a food anti-oxidant and/or nutraceutical composition comprising proline and/or derivatives and/or functional analogues thereof, and/or protein and/or peptide rich in said proline and/or derivatives and/or functional analogues thereof.

The invention also teaches a method to protect the upper digestive tract (oral cavity, oesophagus), stomach and intestines against oxidative damage comprising oral administration of food comprising said food anti-oxidant and/or neutraceutical. A higher intake of said proline and/or derivatives and/or functional analogues thereof, and/or protein and/or peptide rich in said proline and/or derivatives and/or functional analogues thereof may lead to a better protection against damage by ROS in the tissues of the body.

Proline and proline rich proteins or peptides are water soluble, therefore, a medicament comprising proline or a functional derivative thereof to protect cells and tissues against ROS can also be administered intravenously or subcutaneously, or by inhalation to individuals or animals, prior to treatment with ROS inducing methods, like for example, but not limited to: irradiation in cancer treatment, chemotherapy, or photodynamic therapy.

## Detailed description

The dramatic increase of proline in plants occurs over several hours or days after application of the stress. Most of the proline in plants is accumulated in the cytoplasm rather than in vacuoles. The accumulation and protective effect of proline has been observed in many higher plants and bacteria as well as in protozoa, algae, marine invertebrates and insects (2). Stresses such as cold, heat, salt, drought, UV or heavy metal stress cause a significant increase of the proline concentration in a variety of plants. Interpretations of proline accumulation vary from its role as a useful adaptive response, helping organisms to withstand the effect of stress, to merely a consequence of stress induced damage to the cells (3). Transgenic plants, which are not able to produce proline, have a significantly lower stress tolerance (4).

The function of proline in stressed plants is often explained by its property as osmolyte, able to balance water stress (5). In addition, other possible positive roles of proline under stress have been proposed with greater or lesser convictions, which include stabilization of proteins, regulation of the cytosolic pH, and regulation of NAD/NADH ratio.

Stress is the result of the sum of damages in all cellular components (lipids, proteins and nucleic acids). All stresses induce the production of reactive oxygen species, especially singlet oxygen and free radicals which are known to break DNA, impair the function of proteins and are responsible for lipid peroxidation. Plants have evolved diverse strategies of acclimatisation and avoidance to cope with adverse environmental conditions. These strategies include accumulation of compatible solutes like for example glycinebetaine, proline, and mannitol.

Reactive oxygen species cause stress to biological systems. Stress induces in turn production of reactive oxygen species. Therefore, a mechanism to interrupt such an autocatalytic process is required. Under normal circumstances, concentrations of oxygen radicals remain low because of the activity of protective enzymes including superoxide dismutase, catalase and ascorbate peroxidase (6). Under stress, accumulation of compatible solutes occurs, in addition to the increase in the activities of detoxifying enzymes. Interestingly, among various compatible solutes, proline has been shown to protect plants against singlet oxygen, and free radical induced damages (7)

As mentioned before, proline accumulates in high amount in several plants under stress. We show here that this accumulation of proline can protect plants against damage by reactive oxygen species.

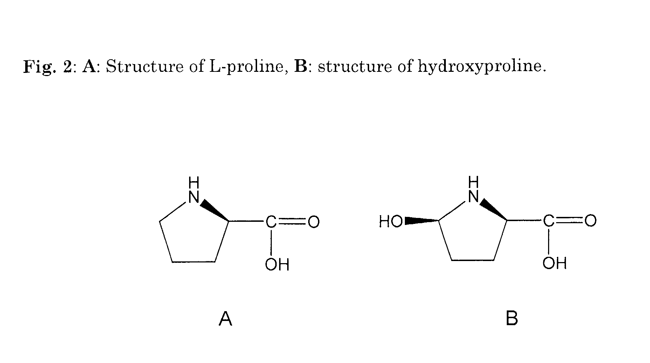

The name of proline has been derived from "pyrrolidine" by Emil Fischer in 1904. The amino acid L-proline (L-pyrrolidine 2-carboxy acid, $C_5H_9NO_2$, Fig. 2A) in its pure form is a colourless substance, highly soluble in water, well soluble (unlike all other amino acids) in alcohols, sparingly soluble in benzene and acetone and insoluble in ether. In aqueous solution at physiological pH the free amino acid proline occurs in its zwitterionic form, carrying negative charge on the deprotonated carboxyl group and positive charge on the doubly protonated amino function. Therefore it also can be referred to as "proline betaine". Due to ring puckering, two conformations of the pyrrolidine ring exist, called *up* and *down.*

Incorporated into peptides, the substituents of the pyrrolidine ring can also assume two conformations, *trans* and *cis,* depending on whether the peptide carbonyl oxygen atoms are pointing to each other *(cis)* or not *(trans).* Proline incorporation has a destabilizing effect on the secondary structure of proteins. As a rotationally hindered secondary amine (unique among all neutral amino acids), proline hardly matches the steric requirements needed for forming an $\alpha$-helix. On the opposite, proline residues are often at the end of an $\alpha$ helix. In the formation of $\beta$ turns, often proline and hydroxyproline ([2S]-[4R]-hydroxy pyrrolidine 2-carbonic acid, Fig. 2B) are involved. In general, $\beta$ turn-rich proteins contain a high amount of (hydroxy)proline, e.g., casein (7 %) and prolamine (20 %). Collagen (26 %) forms a special triple helix.

Primary amino acids R-CH(COOH)(NH$_2$) react chemically as expected from a compound carrying an amino and a carboxyl group. They can be oxidised by thermal, chemical or enzymatic decarboxylation. The products are amides R-CO(NH$_2$), aldehydes R-CH-CHO, and amines R-CH$_2$-NH$_2$. In case of the secondary amino acid proline, the amide is the cyclic 2-pyrrolidone.

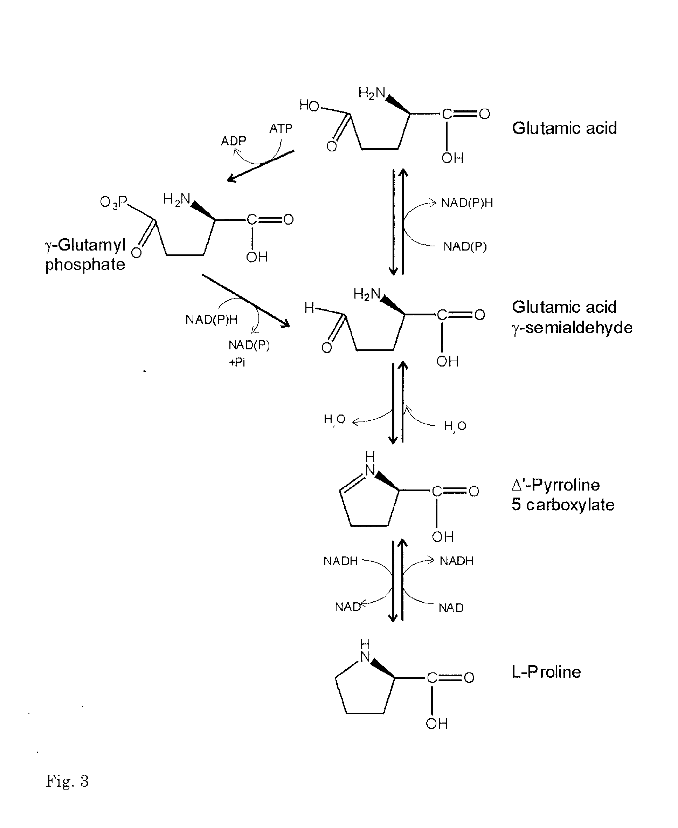

In addition to the reactions similar to the primary amino acids, proline can reversibly perform a ring-opening reaction by addition of a molecule of water, forming glutamic acid $\gamma$-semialdehyde (Fig. 3), a derivative of glutamate, which is the most central amino acid in the network of amino acid biosynthesis. Since the semi-aldehyde is reduced two electron equivalents higher as glutamate, proline can be considered as an "electron rich glutamate", and hence the redox system proline/glutamate provides a mitochondrial "electron sponge" (Fig. 3). In general, proline is formed from glutamate in plants. Alternatively, proline is also enzymatically synthesised from ornithine by exchange of the $\delta$-amino to a $\gamma$-aldehyde group forming the glutamic acid semialdehyde as precursor of proline. Hydroxyproline is produced in the peptide-bound form from proline by oxidation with an ascorbic acid-dependent monooxygenase.

## The protective action of free proline against reactive oxygen species

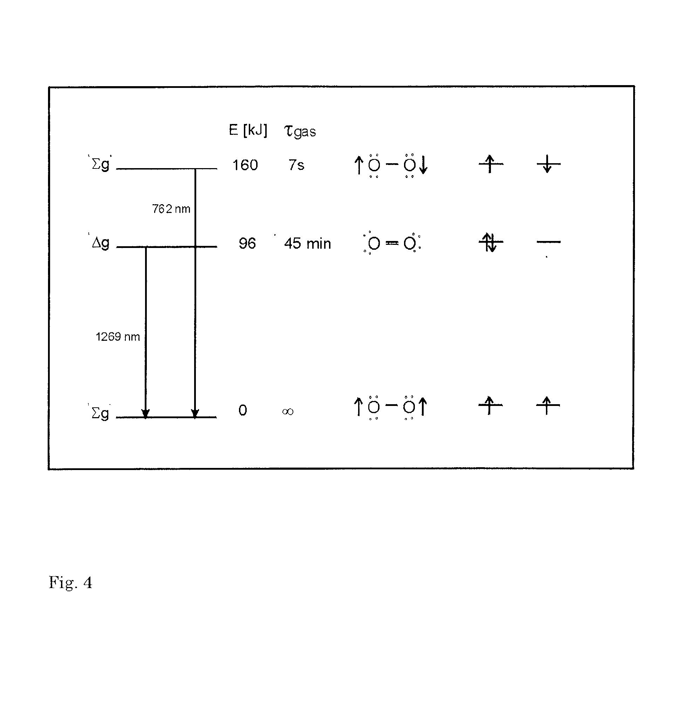

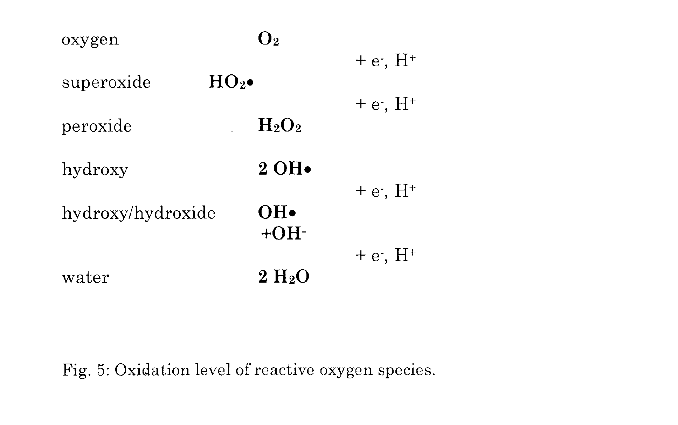

"Normal" molecular oxygen ($^3O_2$) is in its electronic ground state, which is a triplet state. Due to spin-conservation rules, triplet oxygen is rather inactive to biological material. The electronically excited singlet oxygen ($^1O_2$) (Fig. 4) however is highly reactive and rapidly oxidises amino acids, lipids and DNA. Free radicals produced by reaction with $^3O_2$ react similarly aggressively. The one-electron reduced superoxide radical (HO$_2\bullet$ or O$_2^{1-\bullet}$ ) and the hydroxyl radical (OH$\bullet$ ), which is on the same redox level as the peroxide, are the main oxygen radical compounds (Fig. 5). Also the NO$\bullet$ radical, found recently as active component in several biological systems, can cause unwanted damages. In the following, the chemical reactivity of proline with reactive oxygen species is discussed with the aim to understand the molecular mechanism of the protective effect of proline under stress in plants.

## Proline quenches singlet oxygen

First some important properties of singlet oxygen will be introduced. Subsequently, the electronic state of singlet oxygen, its production *(in vitro* and in plants) and its chemical reactivity with proline and other amines are discussed.

### *Electronic state of singlet oxygen*

Since chemical properties depend on the electronic structure, electronically excited molecules undergo different chemical reactions as the same molecules in their electronic ground state. Therefore, molecules in different electronic states have to be regarded as different chemical species. Most molecules have a singlet state (S) as electronic ground state, i.e., all electrons are paired and there is no electronic spin. There are, however, important exceptions. One of them is molecular oxygen (O$_2$) which has triplet state (T) as electronic ground state. "Normal" molecular oxygen (Fig. 4), which is in the air we are breathing, is therefore in triplet state ($^3O_2$) (denoted as $^1\Sigma g^-$). Other examples for compounds with T-ground state are given by carbene compounds (CR$_2$). Due to quantum mechanical spin-conservation rules, chemical reactions between species in triplet and singlet states are spin forbidden, and therefore have a high kinetic reaction barrier. This is the reason why atmospheric oxygen does not react easily with organic matter, although such reaction would be energetically very favourable. In the electronically excited state (denoted as $^1\Delta g$), oxygen becomes an electronic singlet (Fig. 4). Reactions of singlet oxygen with organic molecules are not spin forbidden, and have much less activation energy. One way to circumvent the spin-dependent selection rules is provided by catalysis with transition metals (via spin-orbit coupling of their d-orbitals). Lifetime and action radius of singlet oxygen depend on the surrounding medium. In aqueous solution singlet oxygen has a lifetime of ca. 3 $\mu$s and can diffuse almost 0.2 $\mu$m. This is a rather long lifetime, as compared to other reactive oxygen species. Consequently, chemical reactions of singlet oxygen are much more specific as compared to OH• radicals. Singlet oxygen reacting with DNA interacts selectively with guanine, since it has the lowest redox potential among the nucleotides constituting DNA.

### Production of singlet oxygen

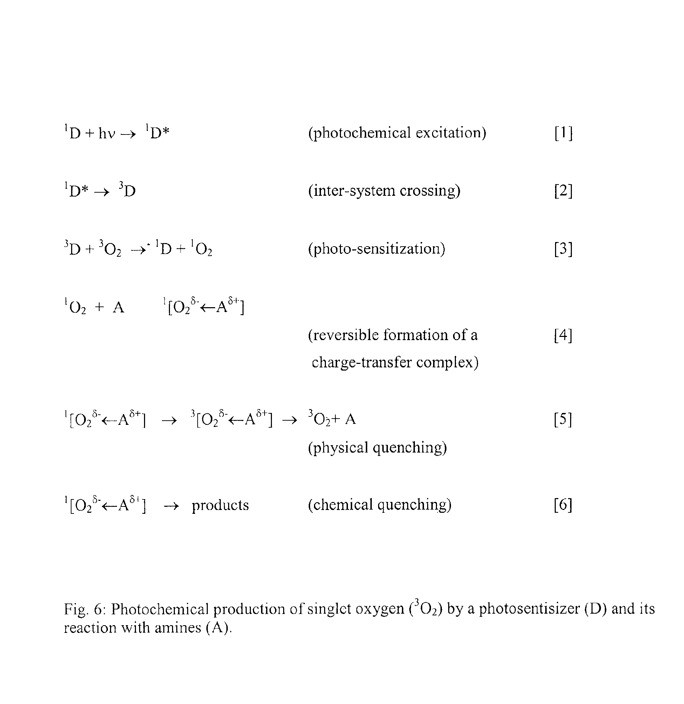

The usual way to produce singlet oxygen *in vitro* is by irradiation of photosensitisers. These are dyes with high quantum yield for triplet formation (Fig. 6, formulae [1] - [3]). In nature singlet oxygen is produced in the same photochemical way. For example $P_{680}$, the primary electron donor in reaction centres of photosystem II in plants, acts as photosensitiser and is the main source of singlet oxygen in plants. Another cellular source of singlet oxygen are heme proteins such as peroxidases. In plants under high light irradiation, the likelihood for the production of singlet oxygen increases, because photons are absorbed faster than electrons are pumped. Singlet oxygen is directly involved in photobleaching of photosynthetic pigments, D1 protein degradation and protein cross-linking (8). Also various stress conditions produce singlet oxygen, which affect the structural integrity of the photosystem II membrane protein complex, and destabilise proteins and membranes further.

### Chemical reactivity of singlet oxygen with proline

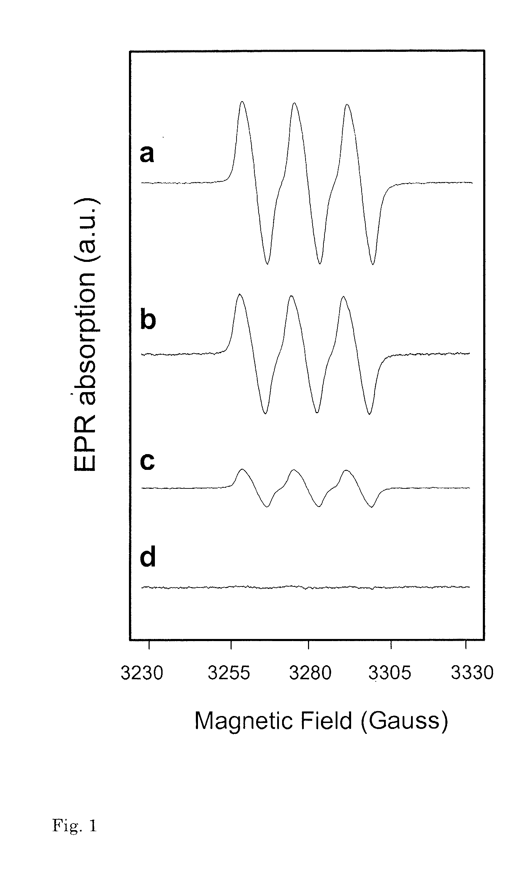

Our recent experiments (9) in an artificial system using toluidine blue as a sensitiser solution showed that proline is an excellent quencher for singlet oxygen in vitro (Fig. 1). Fig. 1a shows the production of singlet oxygen as detected by the formation of 2,2,6,6-tetramethylpiperidine-1-oxyl (TEMPO) by EPR spectrometry. Interestingly, the production of TEMPO was decreased in the presence of 5 and 10 mM proline (Fig. 1b & c) and completely abolished by the presence of proline at concentration as low as 20 mM (Fig. 1d). These results show that proline is a very effective singlet oxygen quencher. This is in line with the low ionisation potential (IP) of proline as discussed below. Glycine or different types of sugar have no effect on the singlet oxygen concentration. In the following paragraphs, the molecular mechanism of the chemical reaction of proline with singlet oxygen is discussed in detail.

The most characteristic feature of the chemical reactivity of singlet oxygen is its electrophilicity. Therefore, C=C or C=O double bonds as well as the lone pairs of sulphur and amines compounds are preferably the targets of singlet oxygen attack. Investigations of the reaction with amines have revealed that the kinetics of the reaction is controlled by the IP (i.e. the capability to provide an electron) of the amine. Therefore, the formation of a charge-transfer (CT) complex in a reversible reaction is assumed to be the initial step (Fig. 6, formula [4]).

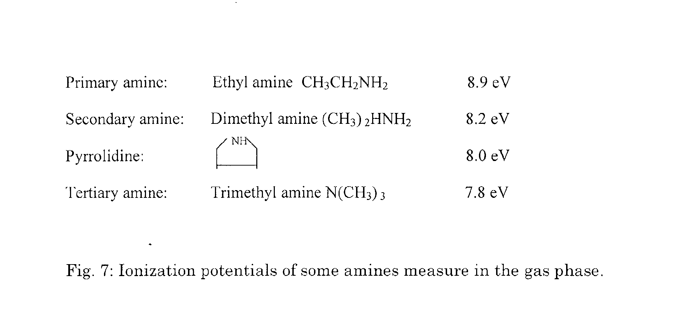

An amine, having a low IP, is easier capable to form a CT complex, and can therefore quench singlet oxygen faster. IPs can be determined either by photoelectron spectroscopy (PES) or electrochemically. The first electron removed from an amine can be assigned to the lone pair of the nitrogen atom. The value of the IP depends on the chemical structure of the amine. Due to the electron-donating character of alkyl substituents, tertiary amines have a lower IP than secondary amines, and secondary amines have a lower IP than primary amines. Therefore, charge transfer complexes of tertiary amines are more stable than secondary and secondary more than primary amines (Fig. 7). Also cyclic compounds are easier to ionise as open-chained amines. The larger the ring, the lower is the IP. The IP of enamines is lower than the IP of tertiary amines. IPs of peptide-bound amino acids are similar to that of isolated amino acids, since interactions among amino and carboxyl end groups are weak.

Pyrrolidine, which forms the 5-membered ring of proline, has a remarkable low IP of 8.0 eV. The substitution of the carboxyl group increases the IP slightly by withdrawing electrons from the ring. The decay of the initial CT complex of the amine with the singlet oxygen can occur either by physical (Fig. 6, formula [5]) or chemical (Formula [6]) quenching. For amines, both pathways are competing. Due to its low IP, proline forms CT complexes with singlet oxygen, and due to its structure, both, chemical and physical quenching is possible. Physical quenching works via a mechanism involving spin-orbit coupling restoring the original amine compound in its singlet ground state. For instance, azide ($N_3$-) is known to quench via the physical pathway. In the case of chemical quenching, the structure of the amine is changed, and a reaction product is formed. One crucial aspect for the feasibility of a chemical pathway is the existence of $\alpha$-hydrogen atoms and the possibility to form a $C=NH^+$ group. For highly constrained amines as 2,2,6,6-tetramethylpiperidine (TEMP), also another chemical pathway, producing stable nitroxide radicals, is possible. This chemical reaction is used for the detection of singlet oxygen by EPR spectroscopy (see above). Hence, the experimental finding of the high capability of proline to quench singlet oxygen can be well understood by its chemical properties.

### Reactivity of proline toward hydroxyl radicals

Compared to singlet oxygen, OH• radicals react much faster and therefore with less selectivity. Both reactive species can react as strong oxidants, too, and are able to abstract hydrogen atoms. The involvement of hydroxyl OH• and superoxide $O_2$-• radicals in oxidative stress is well known. The nitrosyl NO• radical is known to occur in biological systems and also can induce damages.

### Formation of hydroxyl radicals

OH• radicals can be formed in several ways. Fenton's reaction for production of hydroxyl radicals by oxidation of $Fe^{2+}$ ions is well known:

$$Fe^{2+} + H_2O_2 \rightarrow Fe^{3+} + OH\bullet + OH^- .$$

Also other metal ions as $Cu^{1+}$ are able to reduce hydrogen peroxide ($H_2O_2$). In the presence of primary carbonate $HCO_3$-, also $Mn^{2+}$, which occurs in high concentration in plants, can cause the disproportionation of hydrogen peroxide. Hydrogen peroxide also can form two OH• radicals either by heating or by photolyzation under UV irradiation. Organic peroxides as tertiary butyl hydroperoxide provide OH• radicals. Heme proteins undergoing oxygen-redox chemistry can release free radicals. Another method for the production of OH• radicals (together with H• radicals and solvated electrons $e^-aq$) are $\gamma$-rays from a cobalt-60 source. Superoxide radicals are formed by the reaction of H• or $e^-_{aq}$ with molecular oxygen

### Reactions of hydroxyl radicals with proline

Smirnoff and Cumbes (10) have assessed the hydroxyl radical scavenging activity of various compatible solutes including proline which accumulate in plants under stress, and found that sorbitol, mannitol, myo-inositol and proline are effective hydroxyl radical scavengers. Proline reacts with OH• under hydrogen abstraction by forming the most stable radical, which carries the spin on the C-5 atom since it is far from the carboxyl group and close to the nitrogen. In case of hydroxyproline, the radical with spin localisation on the C-4 is more stable.

Most of the OH• radicals generated *in vivo*, except during excessive exposure to ionising radiation, comes from Fenton's reaction by oxidation of metals. Whether this reaction occurs and OH• radicals are released, depends on the state of complexation of the redox-active metal. Normally, metal ions in aqueous solution do not exist "naked" but are either hydrated or ligated by other molecules such as hydrogen carbonate or chelating compounds as EDTA, which have a strong effect on the chemical reactivity of the metal. Oxygen rapidly oxidises $Fe(OH)_2$ to Fe(III). Because at physiological pH, Fe(III) exists largely as insoluble polymeric $Fe(OH)_3$, sub-stoichiometrical amounts of chelating compounds can keep a trace of iron soluble, catalysing Fenton's reaction. Under these conditions, the reaction may occur in the complexation shell of the redox-active metal and may only affect the ligands. Such a "caged reaction" can effectively protect the surrounding biological material since the OH• radical cannot attack outside the cage. This means that binding of proline to redox-active metal ions can protect the surrounding biological tissue from damage by OH• radicals.

### Proline residues in proteins

### Singlet oxygen quenching

Proline incorporated into a peptide backbone becomes a tertiary amide. The IP of peptide-bound proline residues is similar to free proline, and therefore its capability to quench singlet oxygen is also similar. It has been shown, that proline-rich proteins, stabilising cell walls, are secreted by salt-adapted bean cells. Also in animal and human cells, production of proline-rich proteins has been described. Examples are the salivary proline-rich proteins (PrP's)(11) and the epithelial small proline-rich proteins (SPRR's)(12). SPRR's are evolutionary related to late envelope proteins (LEP's) with which they share a conserved proline-rich domain.The three classes of proteins can be crosslinked by transglutaminases. PrP's are part of the salivary pellicle covering the tooth surface, whereas SPRR's (also named SPR's, cornifins or pancornulins) and LEP's are precursor proteins of the cornified cell envelope of the epidermal skin and internal squamous epithelia (lining of oral cavity, oesophagus, cervix and vaginal epithelium)(13). Interestingly, the expression of SPRR's is strongly induced after exposure of epidermal keratinocytes (14) or skin (15) to UV radiation.

Recently, it was shown that SPRR proteins are also strongly induced in axotomized neurons and play a role during nerve regeneration. We foresee a possible role of SPRR's as anti-oxidants in this process, where degeneration of the distal portion of the nerve is tightly linked with massive cell growth at the proximal site. As a matter of fact, the brain is especially sensitive to oxidative damage because of its high content of readily oxidized fatty acids and high use of oxygen.

### Hydroxyl radical quenching

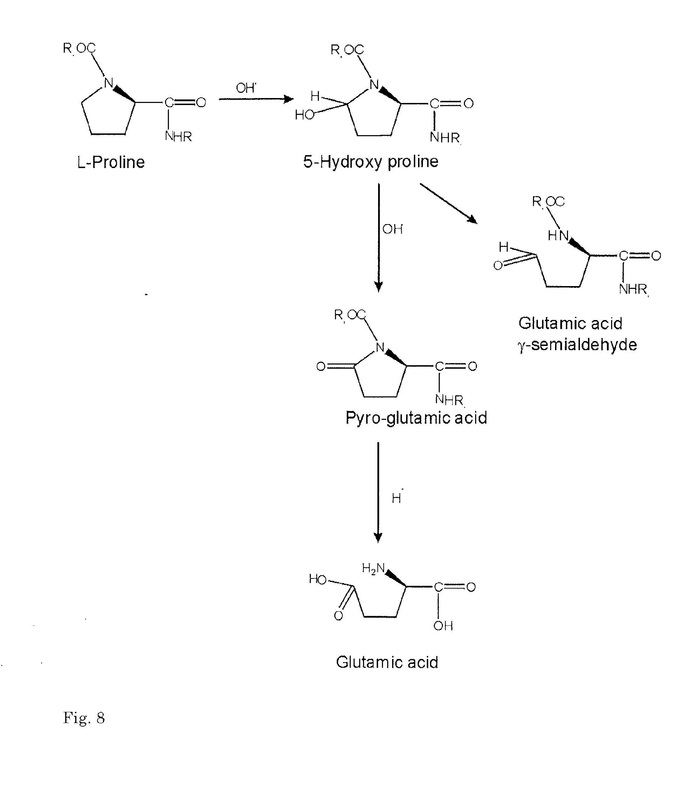

Attack by OH• and $HO_2\bullet$ radicals is known to destabilise proteins. The peptide backbone can be cleaved by forming a carbon centred radical after $\alpha$-hydrogen abstraction. Furthermore, side chains can be attacked. Proline residues can be preferred sites of radical attack. Proline can be oxidised to various compounds (Fig. 8). Formation of 4-hydroxyproline will not cause a cleavage of the polypeptide. Also the formation of 5-hydroxyproline, which can hydrolyse to glutamate $\gamma$-semialdehyde, does not damage the amino-acid backbone. The observation of $\gamma$-aminobutyric acid, glutamic acid, and 2-pyrrolidone, however, has to be explained by backbone cleavage. Oxidation of proline to 2-pyrrolidone provides a unique mechanism for peptide backbone cleavage that is not associated with the formation of a reactive carbonyl group. The very high content of proline and hydroxyproline in collagen is not always advantageous for the maintenance of tissue structures under metabolic conditions. Collagen degradation is a hallmark of photodamaged connective tissue. X-ray irradiation, applied in lung cancer, is known to induce fibrosis, i.e. an excessive accumulation of collagen, limiting the irradiation level in radiation therapy of thoracic tumours.

### Caged reactions in the enzyme

If the redox-active metal is bound to the protein, the oxidation can be site specific. The Fenton-type generation of the OH• radical, is followed by the formation of a more stable carbon-centred radical. Similar as guanine becomes selectively oxidised within nucleic acids, "hole hopping" may also occur among amino acids, forming the most stable amino-acid radical. Such "caged" enzymatic reactions can be important for the function of an enzyme, and several proteins with oxidative modified amino acids in the reaction centre are known. Cross-linked amino acid residues are known (e. g. the reactive centres of cytochrome-c oxidase and galactose oxidase. In both cases, the two covalently bound amino acids are functionally important and are in direct neighbourhood of the metal. Site-directed oxidation of proline, histidine, arginine, lysine, threonine, tyrosine and cysteine has been reported (16). Hence, proline residues, which are very good singlet oxygen quenchers and traps for OH• radicals, stabilise proteins endogenously.

The high capability of proline to quench singlet oxygen and hydroxyl radicals can be well understood by its chemical properties. Pyrrolidine, which forms the 5-membered ring of proline, has a remarkable low IP, and therefore proline is capable of forming a charge transfer complex and can quench singlet oxygen effectively. Proline reacts with OH• under hydrogen abstraction by forming the most stable radical, which carries the spin on the C-5 atom. Therefore, proline accumulation in high amounts in plants under stress could be well understood by its property to scavenge reactive oxygen species. Genetic engineering to enhance proline biosynthesis in important crop plants, which do not accumulate proline under stress, may be an important strategy to tackle the high level of reactive oxygen species generated during stressful conditions. Furthermore, dermatology may profit from understanding the action of proline-rich proteins in order to protect tissues from radiation-induced damages.

### Examples

### 1. EPR on in-vitro systems

We use electron paramagnetic resonance (EPR) to demonstrate the excellent singlet-oxygen quenching properties of proline in chemical sensitiser solutions. Experiments were also performed with a purified small proline rich protein (SPRR4, 16.4% proline) and showed a quenching effect, which on a molar ratio was comparable to that of free proline. These experiments were repeated with different protein samples (various members of the SPRR family having up to 39.7% proline and other unrelated proteins with low proline content), in order to quantify the quenching properties of protein-bound proline. The synergistic effect of clustered proline residues in a protein is also studied.

### 2. Cell survival under stress

Survival rates of various cell lines expressing high amounts of different SPRR proteins are determined after application of singlet-oxygen stress or other forms of stress. The protecting effect of varying proline concentrations in the culture medium is also analysed.

### 3. In-vivo MRI on mice

Mice are fed with $^{13}$C-isotope and/or $^{15}$N labelled proline to follow the proline metabolism and incorporation in real time and with spatial resolution in living organisms by magnet-resonance imaging (MRI). Due to different NMR chemical shift, proline and its metabolisation products are distinguished.

### FIGURE LEGENDS:

**Fig. 1:** *In-vitro* quenching of singlet oxygen by proline: Nitroxide radical (TEMPO) were formed due to reaction of sterically hindered amine (TEMP) with singlet oxygen ($^1O_2$) generated by photoirradiation of toluidine blue in the absence (a) or presence of 5 mM (b), 10 mM (c) and 20 mM (d) proline. The sample contained 1 mM toluidine blue and 10 mM TEMP was irradiated with white light (1200 µE m$^{-2}$sec$^{-1}$) for 20 min. EPR spectra were recorded with an X-band EPR spectrometer at modulation amplitude 1 Gauss; modulation frequency 100 kHz; microwave power 15 mW; temperature 25°C.

**Fig. 2:** Structure of L-proline, **B:** Structure of hydroxyproline.

**Fig. 3:** Proline biosynthesis and oxidation showing ring opening from proline to glutamate and proline-glutamate electron sponge system

**Fig. 4:** Structure and life time (τ) of electronically excited oxygen molecules. The $^1\Sigma$g- ground state is a triplet. The two singlet states $^1\Delta$g and $^1\Sigma$g$^+$ are electronically excited. (Adapted from W. Adam (1981) Chem. Unsere Zeit 15, 190-196)

**Fig. 5:** Oxidation level of reactive oxygen species.

**Fig. 6:** Photochemical production of singlet oxygen ($^3O_2$) by a photosentisizer (D) and its reaction with amines (A).

**Fig. 7:** Ionization potentials of some amines measure in the gas phase.

**Fig. 8:** Proline residues in polypeptide/proteins. The prolyl residues are selectively oxidised by OH° to glutamic acid.

## References

1. Alia, Pardha Saradhi, P., Mohanty, P. (1997) J. Photochem. Photobiol. B: 38, 253-257.
2. Delauney, A.J., Verma, D.P.S. (1993) Plant J. 4, 215-223.
3. Anjum, F., Rishi, V., Ahmed, F. (2000) Biochim. Biophys. Acta, 1476, 75-84.
4. Xin, Z., Browse, J. (1998) Proc. Natl. Acad. Sci. USA 95, 7799-7804.
5. Chang, Y.C., Lee, T.M. (1999) Bot. Bul. Acad. Sinica 40, 289-294.
6. Asada, A. (1984) Meth. Enzymol. 105, 422-428.
7. Alia, Saradhi, P.P. and Mohanty, P. (1991) Biochem. Biophys. Res. Comm. 181, 1238-1244.
8. Mishra, N.P., Franke, C., Van Gorkom, H.J., Ghanotakis, D.F. (1994) Biochim. Biophys. Acta 1186, 81-90.
9. Alia, Mohanty, P., Matysik, J. (2001) Amino Acids 21, 195-200.
10. Smirnoff, N., Cumbes, Q.J. (1989) Phytochem. 28, 1057-1060.
11. Bennick, A. (1982). Mol. Cell Biochem. 45, 83-99.
12. Kartasova, T., van de Putte, P. (1988) Mol. Cell Biol. 8, 2195-2203.
13. Backendorf, C., Hohl, D. (1992) Nat. Genet. 2, 91.
14. Cabral, A., Voskamp, P., Cleton-Jansen, A.M., South, A., Nizetic, D., Backendorf, C. (2001) J. Biol. Chem. 276, 19231-19237.
15. Yaar, M., Eller, M. S., Bhawan, J., Harkness, D. D., DiBenedetto, P. J., Gilchrest, B. A. (1995) Exp. Cell Res. 217, 217-226.
16. Stadtman, E.R. (1993) Annu. Rev. Biochem. 62, 797-821.

## Claims

1. Use of proline and/or functional derivatives thereof, and/or protein and/or peptide rich in said proline and/or functional derivatives thereof for quenching radicals.

2. Use according to claim 1 wherein said radicals comprise reactive oxygen species (ROS).

3. Use of proline and/or functional derivatives thereof, and/or protein and/or peptide rich in said proline and/or functional derivatives thereof for preventing and/or diminishing damage by ROS.

4. Use according to claim 3, wherein additional proline and/or functional derivatives thereof, and/or protein and/or peptide rich in said proline and/or functional derivatives thereof is provided for preventing damage to cells and tissue.

5. Use of proline and/or functional derivatives thereof, and/or protein and/or peptide rich in said proline and/or functional derivatives thereof for the preparation of a medicament for diminishing or preventing damage to cells and/or tissues by ROS.

6. Use according to any one of claims 1-3 wherein said proline and/or functional derivatives thereof, and/or protein and/or peptide rich in said proline and/or functional derivatives thereof is added to a food substance.

7. Use according to any one of claims 1-4 wherein said proline and/or functional derivatives thereof, and/or protein and/or peptide rich in said proline and/or functional derivatives thereof is added to a topical composition.

8. Use according to any one of claims 1-3 wherein said proline and/or functional derivatives thereof, and/or protein and/or peptide rich in said proline and/or functional derivatives thereof is added to a pharmaceutical composition.

9. A topical composition comprising proline and/or derivatives thereof, and/or protein and/or peptide rich in said proline and/or derivatives thereof and a suitable carrier or diluent.

10. A method to prevent excessive tanning comprising applying a composition according to claim 9, prior to exposure of the body to a ROS inducing condition, for example radiation.

11. A method to protect hair, eyes, nails, skin and teeth against damage by ROS comprising applying a composition according to claim 9, prior to exposure of the body to a ROS inducing condition, for example dyeing, bleaching, or cleaning.

12. A pharmaceutical composition comprising a proline-rich proteinaceous molecule of the gene family of proline-rich proteins (PrP's) and/or small proline-rich proteins (SPRR) and/or Late Envelope Proteins (LEP) and/or collagen, and/or casein, and/or elastin and/or muscle protein such as myosin, and a suitable carrier or diluent.

13. A method to protect cells and tissues of a mammal against damage by ROS by administration of a pharmaceutical composition according to claim 12 prior to a ROS inducing treatment or diagnosis.

14. The use of proline and/or functional derivatives and/or functional analogues thereof, and/or protein and/or peptide rich in said proline and/or functional derivatives thereof for the preparation of a food antioxidant and/or a nutraceutical composition.

15. A method to protect mammals against oxidative damage comprising increasing the proline level of their food by adding proline and/or functional derivatives and/or functional analogues thereof, and/or protein and/or peptide rich in said proline and/or functional derivatives and/or functional analogues thereof.

Fig. 1

Fig. 2: **A**: Structure of L-proline, **B**: structure of hydroxyproline.

A                                    B

Fig. 3

Fig. 4

oxygen            $O_2$

$+\ e^-,\ H^+$

superoxide      $HO_2\bullet$

$+\ e^-,\ H^+$

peroxide          $H_2O_2$

hydroxy          $2\ OH\bullet$

$+\ e^-,\ H^+$

hydroxy/hydroxide    $OH\bullet$
                     $+OH^-$

$+\ e^-,\ H^+$

water            $2\ H_2O$

Fig. 5: Oxidation level of reactive oxygen species.

$^1D + h\nu \rightarrow {}^1D*$ (photochemical excitation) [1]

$^1D* \rightarrow {}^3D$ (inter-system crossing) [2]

$^3D + {}^3O_2 \rightarrow {}^1D + {}^1O_2$ (photo-sensitization) [3]

$^1O_2 + A \quad {}^1[O_2{}^{\delta-}{\leftarrow}A^{\delta+}]$

(reversible formation of a [4]
charge-transfer complex)

$^1[O_2{}^{\delta-}{\leftarrow}A^{\delta+}] \rightarrow {}^3[O_2{}^{\delta-}{\leftarrow}A^{\delta+}] \rightarrow {}^3O_2 + A$ [5]
(physical quenching)

$^1[O_2{}^{\delta-}{\leftarrow}A^{\delta+}] \rightarrow$ products (chemical quenching) [6]

Fig. 6: Photochemical production of singlet oxygen ($^3O_2$) by a photosentisizer (D) and its reaction with amines (A).

| | | |
|---|---|---|
| Primary amine: | Ethyl amine $CH_3CH_2NH_2$ | 8.9 eV |
| Secondary amine: | Dimethyl amine $(CH_3)_2HNH_2$ | 8.2 eV |
| Pyrrolidine: | | 8.0 eV |
| Tertiary amine: | Trimethyl amine $N(CH_3)_3$ | 7.8 eV |

Fig. 7: Ionization potentials of some amines measure in the gas phase.

Fig. 8

**European Patent Office**

## PARTIAL EUROPEAN SEARCH REPORT

**Application Number**

which under Rule 45 of the European Patent Convention EP 02 07 5964
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 715 850 A (JAPAN RES DEV CORP ;MORI MASATO (JP); TORII KUNIO (JP)) 12 June 1996 (1996-06-12) * claim 1 * | 1-3,6-8 | A61K31/401 A61K38/00 A61K38/39 A61K38/17 A61K7/48 |
| X | US 6 187 743 B1 (OBI-TABOT ELIOT T) 13 February 2001 (2001-02-13) * claims 28,29 * | 1-3,6-8, 12 | A23L3/34 A61P1/00 A61P39/06 A61P17/00 |
| X | WO 00 18924 A (INCYTE PHARMA INC ;CORLEY NEIL C (US); REDDY ROOPA (US); BAUGHN MA) 6 April 2000 (2000-04-06) * page 23, line 11-16,26,33,34 * * page 29, line 6,7,12,15-18 * * claims 19,20 * | 1-5,7-9, 11-13,15 | |
| X | EP 0 308 278 A (GIVAUDAN LAVIROTTE) 22 March 1989 (1989-03-22) * claims 1-5 * | 1-6,14, 15 | |
| X | WO 95 27408 A (EAST WELLSUM IND S PTE LTD ;ZENGSHEN JIA (CN)) 19 October 1995 (1995-10-19) * claims 1,2 * | 1-5,7, 9-11 | **TECHNICAL FIELDS SEARCHED (Int.Cl.7)** A61K A23L |

-/--

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 30 July 2002 | Lechner, O |

Although claims 1-4, 6-8 (as fas as relating to an in vivo use), 13 and 15 are directed to a method of treatment of the human/animal body (Article 52(4) EPC), the search has been carried out and based on the alleged effects of the compound/composition.

_____

Claim(s) searched incompletely:
      1-15

Reason for the limitation of the search:

Present claims 1-15 relate to an extremely large number of possible molecules, i.e. functional proline derivatives, proteins/peptides rich in proline and proline rich proteins, functional derivatives thereof and similar expressions. In fact, the claims contain so many options that a lack of clarity (and conciseness) within the meaning of Article 84 EPC arises to such an extent as to render a meaningful search of the claims impossible. Consequently, the search has been carried out for those parts of the application which do appear to be clear (and concise), namely proline, collagen, casein, elastin, SPRRs and late envelop proteins.

As far as relating to an in vivo use, present claims 1-4, 6-8, 11, 13 and 15 relate to an extremely large number of ill defined pathologic conditions/diseases circumscribed by a physiochemical/biochemical process, i.e. quenching radicals, oxidative damage, damage by ROS, damage to cells and tissue, damage to cells and tissue by ROS, a ROS inducing treatment/diagnosis/condition etc. In fact, the claims contain so many options, that a lack of clarity (and conciseness) within the meaning of Article 84 EPC arises to such an extent as to render a meaningful search of the claims impossible. Consequently, the search has been carried out for those parts of the claims which appear to be supported and disclosed, namely those parts relating to well defined pathologic conditions/diseases mentioned in the application, i.e. stomach ulcers, duodenal ulcers and Crohn's disease (c.f. page 4, line 25-27).

**European Patent Office**

**PARTIAL EUROPEAN SEARCH REPORT**

Application Number

EP 02 07 5964

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| A,D | ALIA ET AL: "Effect of proline on the production of singlet oxygen." AMINO ACIDS (VIENNA), vol. 21, no. 2, 2001, pages 195-200, XP001086573 ISSN: 0939-4451 * abstract * | 1-15 | |
| A | DEUSCHLE KAREN ET AL: "A nuclear gene encoding mitochondrial DELTA1-pyrroline-5-carboxylate dehydrogenase and its potential role in protection from proline toxicity." PLANT JOURNAL, vol. 27, no. 4, August 2001 (2001-08), pages 345-355, XP001086572 ISSN: 0960-7412 * page 346, left-hand column, paragraphs 3,4 * * page 345, left-hand column * * abstract * | 1-15 | **TECHNICAL FIELDS SEARCHED** (Int.Cl.7) |
| A | VENOT CORINNE ET AL: "The requirement for the p53 proline-rich functional domain for mediation of apoptosis is correlated with specific PIG3 gene transactivation and with transcriptional repression." EMBO (EUROPEAN MOLECULAR BIOLOGY ORGANIZATION) JOURNAL, vol. 17, no. 16, 17 August 1998 (1998-08-17), pages 4668-4679, XP002205372 ISSN: 0261-4189 * page 4669, left-hand column, paragraph 3 * * abstract * | 1-15 | |

-/--

EPO FORM 1503 03.82 (P04C10)

EP 1 342 474 A1

**European Patent Office** **PARTIAL EUROPEAN SEARCH REPORT** **Application Number**

EP 02 07 5964

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| A | SOSZYNSKI MIROSLAW ET AL: "Effect of amino acid peroxides on the erythrocyte." FREE RADICAL BIOLOGY & MEDICINE, vol. 20, no. 1, 1996, pages 45-51, XP002205373 ISSN: 0891-5849 * page 50, right-hand column * * page 45, left-hand column, paragraph 1 * * abstract * --- | 1-15 | |
| A | DONALD STEVEN P ET AL: "Proline oxidase, encoded by p53-induced gene-6, catalyzes the generation of proline-dependent reactive oxygen species." CANCER RESEARCH, vol. 61, no. 5, 1 March 2001 (2001-03-01), pages 1810-1815, XP002205374 ISSN: 0008-5472 * abstract * ----- | 1-15 | **TECHNICAL FIELDS SEARCHED** (Int.Cl.7) |

EPO FORM 1503 03.82 (P04C10)

21

## ANNEX TO THE EUROPEAN SEARCH REPORT
## ON EUROPEAN PATENT APPLICATION NO.

EP 02 07 5964

This annex lists the patent family members relating to the patent documents cited in the above-mentioned European search report.
The members are as contained in the European Patent Office EDP file on
The European Patent Office is in no way liable for these particulars which are merely given for the purpose of information.

30-07-2002

| Patent document cited in search report | | Publication date | Patent family member(s) | | Publication date |
|---|---|---|---|---|---|
| EP 0715850 | A | 12-06-1996 | CA | 2164686 A1 | 08-06-1996 |
| | | | EP | 0715850 A1 | 12-06-1996 |
| | | | JP | 8208472 A | 13-08-1996 |
| US 6187743 | B1 | 13-02-2001 | US | 6046160 A | 04-04-2000 |
| | | | AU | 4191500 A | 13-02-2001 |
| | | | BR | 0013038 A | 30-04-2002 |
| | | | EP | 1206271 A1 | 22-05-2002 |
| | | | WO | 0107071 A1 | 01-02-2001 |
| WO 0018924 | A | 06-04-2000 | AU | 6507299 A | 17-04-2000 |
| | | | WO | 0018924 A1 | 06-04-2000 |
| EP 0308278 | A | 22-03-1989 | FR | 2619711 A1 | 03-03-1989 |
| | | | AT | 71285 T | 15-01-1992 |
| | | | BR | 8804446 A | 28-03-1989 |
| | | | DE | 3867575 D1 | 20-02-1992 |
| | | | DK | 485788 A | 03-03-1989 |
| | | | EP | 0308278 A1 | 22-03-1989 |
| | | | ES | 2033447 T3 | 16-03-1993 |
| | | | JP | 1131107 A | 24-05-1989 |
| | | | KR | 9101912 B1 | 30-03-1991 |
| | | | PT | 88398 A | 31-07-1989 |
| WO 9527408 | A | 19-10-1995 | CN | 1110112 A | 18-10-1995 |
| | | | GB | 2292314 A | 21-02-1996 |
| | | | WO | 9527408 A1 | 19-10-1995 |
| | | | AU | 2081795 A | 30-10-1995 |

EPO FORM P0459

For more details about this annex : see Official Journal of the European Patent Office, No. 12/82